# EUROPEAN PATENT APPLICATION

(11) **EP 1 576 941 A1**
(43) Date of publication of application: **21.09.2005**
(21) Application number: 03780911.8
(22) Date of filing: 18.12.2003
(51) Int. Cl.: A61F 9/007

(54) **AQUEOUS HUMOR DRAINAGE IMPLANT FOR TREATING GLAUCOMA**

(30) Priority: 27.12.2002 JP 2002381239
(71) Applicant: Japan Science and Technology Corporation, Saitama 332-0012 (JP)
(72) Inventor: TAKASHIMA, Seisuke, Kurashiki-shi, Okayama 710-0025 (JP); MORIZANE, Yuki, Okayama-shi, Okayama 700-0971 (JP)
(74) Representative: Bentz, Jean-Paul
(86) International application number: PCT/JP2003/016284
(87) International publication number: WO 2004/060219

(57) **Abstract**

A first tube (3) and second tube (7) for guiding aqueous humor to the exterior of the eye are connected to each other in the vicinity of a surface of conjunctiva (14) via a first joint (5) and second joint (6). A filter part (9) provided to prevent reflux infection from the exterior to interior of the eye is connected to the second tube (7) via a third tube (8) positioned inside a lower lacrimal canaliculus (23). This enables an aqueous humor drainage implant (1) to be positioned in the eye and the exterior of the conjunctiva with reduced invasiveness. With the aqueous humor drainage implant for glaucoma treatment, the aqueous humor in the eye can be drained to the exterior of the conjunctiva while preventing reflux infection at the viral level, and the intraocular pressure reducing effect can be sustained for extended time periods over the lifespan of the patient. Further, the aqueous humor drainage implant can be readily positioned with reduced surgical invasiveness, while posing no danger of damaging the eye or nasolacrimal duct after the installation.

## Description

### TECHNICAL FIELD

The present invention relates to a treating device for effectively draining aqueous humor from the interior of the eye to the exterior of the conjunctiva, used to reduce intraocular pressure in glaucoma or other diseases associated with elevated intraocular pressure.

### BACKGROUND ART

In a normal eye, the aqueous humor produced by the ciliary body circulates through the anterior and posterior chambers before it is drained through the Schlemm's canal and trabecular meshwork providing certain outflow resistance against the drained aqueous humor. Intraocular pressures no greater than 21 mmHg is considered to be in the normal range. Glaucoma is believed to be a consequence of interrupted outflow of aqueous humor through the Schlemm's canal and trabecular meshwork, which occurs either essentially, or secondarily due to inflammations or the like, leading to excess level of aqueous humor in the eye and elevated intraocular pressure. Glaucoma is a disease characterized by damage to the optic nerve caused by elevated intraocular pressure which, if not checked, may lead to a narrowing of the field of vision or visual loss, and eventually to blindness.

Currently, the only way to treat glaucoma is to adjust intraocular pressure. The treatment intends to stop the progress of optic nerve atrophy by lowering intraocular pressure. This is achieved either by suppressing production of aqueous humor or by facilitating outflow of aqueous humor. The treatment is classified into a conservative method and invasive method. The conservative method intends to lower intraocular pressure with use of an eye drops or oral medicine. The invasive method is used when the conservative treatment alone is not sufficient to reduce intraocular pressure. The invasive treatment is given to facilitate outflow of the aqueous humor.

A representative example of the invasive treatment is trabeculectomy. In trabeculectomy, an artificial opening to the anterior chamber is formed through the sclerocornea to provide a drainage for the aqueous humor, and a filtering bleb is formed under the conjunctiva in order to drain the aqueous humor from the anterior chamber to the tissues under the conjunctiva and have these tissues absorb the aqueous humor. However, this method may cause many complications. For example, in the early stage of operation, the trabeculectomy may cause problems associated with excess drainage of aqueous humor, such as hypoplasia of the anterior chamber, choroidal detachment, low intraocular pressure maculopathy, and malignant glaucoma. In the late stage of operation, the trabeculectomy may cause problems associated with wound healing, such as clogging of the aqueous humor drainage, high intraocular pressure due to malabsorption of the aqueous humor caused by fusion of the conjunctiva to the sclera, leakage of the aqueous humor from the filtering bleb, and endophthalmitis.

In the light of such problems of the trabeculectomy, there have been developed numerous aqueous humor drainage devices (aqueous humor drainage implants) implantable to the human body. As does the trabeculectomy, the aqueous humor drainage implant currently in use drains the aqueous humor to the region under the conjunctiva and the aqueous humor is absorbed by the tissues under the conjunctiva. For this purpose, the aqueous humor drainage implant includes a tube that communicates between the interior of the eye and the space under the conjunctiva, and a plate provided in the space under the conjunctiva. That is, the aqueous humor drainage implant is a device for providing a space for absorbing aqueous humor. This is achieved by the tube that prevents the aqueous humor drainage from being clogged, and the plate that prevents fusion between the conjunctiva and sclera.

However, after extended time periods since the operation, there are cases where the underlying tissue of the conjunctiva around the plate fuses together and leaves a scar as a result of a xonobiotic reaction against the aqueous humor drainage implant, or wound healing. In this case, the aqueous humor cannot be absorbed easily, and effective drainage of the aqueous humor cannot be achieved.

In order to overcome such problems of the aqueous humor drainage implant, there have been a number of proposals to drain the aqueous humor from the interior of the eye to the exterior of the conjunctiva in particular.

For example, there has been proposed a method in which aqueous humor is passed to the nasolacrimal duct through a tube (see Patent Document 1, for example). However, owning to the fact that the drainage tube is inserted either directly into the nasolacrimal duct by forming a temporary path in the lacrimal sac, or from the lacrimal canaliculus through the eyelid tissue, the method involves complex procedures and the surgical operation is highly invasive.

The filter used to prevent reflux infection is a flat plate and a Millipore filter is used therefor (the product of Millipore Corporation). The filter is positioned at a stump proximal to the nasolacrimal duct. However, the method involves complex procedures and the surgical operation is highly invasive. Further, depending on the size of the filter, the filter may damage the nasolacrimal duct after installation, yet no specific countermeasure is disclosed as to the problem of filter size.

As to the function of the Millipore filter, a pore diameter range of from 0.1 µm to 10 µm is simply described as being preferable. However, with such a filter function, it would be impossible to prevent reflux infection due to viruses, such as the parvovirus, having a diameter of about 0.02 µm.

There has also been proposed a method in which a tube equipped with a filter is positioned to extend into the exterior of the conjunctiva from the eye, leaving the tube hung in the conjunctival sac (see Patent Document 2, for example). as disclosed in this publication, the filter used to prevent reflux infection is rectangular in shape, and has a polycarbonate filler. Further, as described in the publication, the filter is positioned in the conjunctival sac. However, with the rectangular shape, it is highly likely that the filter will damage the conjunctiva or cornea after the installation. As to the function of the filter, the publication simply describes using a filter with a pore diameter of approximately 0.22 µm for the purpose of preventing entry of bacteria. However, with a pore diameter of approximately 0.22 µm, it would be impossible to prevent reflux infection due to viruses, such as the parvovirus, having a diameter of about 0.02 µm. Further, the publication does not disclose anything about a method of placing the aqueous humor drainage device in the lacrimal canaliculus, lacrimal sac, or nasolacrimal duct.

There has also been proposed a method in which a drainage tube using a hollow fiber membrane is implanted through the sclerocornea so as to drain aqueous humor through the tube placed in the conjunctival sac (see Patent Document 3, for example). However, since the drainage tube (silicon tube) of this aqueous humor drainage device is passed through the sclerocornea, it poses the great danger of causing problems associated with scarring, such as aqueous humor leakage, infection, or damage to the endothelium camerae anterioris.

Further, the drainage tube of the aqueous humor drainage implant has a one-piece structure instead of being provided as multiple tubes, and lacks flexibility. Thus, when placed in the conjunctival sac, the aqueous humor drainage implant may cause conjunctival hermorrhage, allergic reaction, or foreign-body sensation when blinking. As to the filter, the publication discloses using a hollow fiber membrane employing a porous membrane with a pore diameter of approximately 0.005 µm to 0.3 µm.

A drawback of the aqueous humor drainage device disclosed in Patent Document 3, however, is that when the filter function of the hollow fiber membrane is exploited for extended periods of time, clogging occurs in the hollow fiber membrane due to proteins or other substances contained in the aqueous humor, deteriorating permeability of the filter. The reduced filer permeability may lead to increased outflow resistance of the aqueous humor and elevation of intraocular pressure. The publication does not disclose anything about such a possibility or countermeasures for the problem.

Further, because the aqueous humor drainage device disclosed in Patent Document 3 is placed through the cornea, withdrawing and replacing the aqueous humor drainage device is highly invasive. Indeed, it is highly likely that it leads to various complications such as endophthalmitis, corneal astigmatism, and suture failure in the cornea.
[Patent Document 1] U.S. Patent No. 4,886,488 (published on December 12, 1989)
[Patent Document 2] U.S. Patent No. 5,346,464 (published on September 13, 1994)
[Patent Document 3] Japanese Laid-Open Patent Publication No. 117267/1996 (Tokukaihei 8-117267, published on May 14, 1996)

### [Problems to be solved by the invention]

As described above, none of the aqueous humor drainage devices disclosed in the foregoing Patent Documents 1 through 3 can drain aqueous humor to the exterior of the conjunctiva while preventing reflux infection at the viral level, and sustain the intraocular pressure reducing effect for extended time periods over the entire lifespan of the patient. Further, owning to the fact that the aqueous humor drainage devices require a complex procedure for positioning and highly invasive surgical procedures, the devices pose the danger of damaging the eye or nasolacrimal duct after the installation.

The present invention was made in view of the foregoing problems, and an object of the invention is to provide an aqueous humor drainage implant for glaucoma treatment, which can be used with reduced surgical invasiveness and reduced risk of damaging the eye or nasolacrimal duct after the installation, and which can drain aqueous humor to the exterior of the conjunctiva while preventing reflex infection, and sustain the intraocular pressure reducing effect for extended time periods over the lifespan of the patient.

### DISCLOSURE OF INVENTION

The inventors of the present invention diligently worked to solve the foregoing problems and accomplished the invention providing an aqueous humor drainage implant for glaucoma treatment. The aqueous humor drainage implant of the invention can be positioned with reduced invasiveness while preventing damage to the eye or nasolacrimal duct after the installation and at the same time preventing reflux infection. This was achieved by providing an eye-side guiding tube part and an outside-conjunctiva guiding tube part in a guiding tube part used to guide aqueous humor to a filter part positioned externally to the eye, and by connecting the eye-side guiding tube part to the filter part via the outside-conjunctiva guiding tube part.

In order to solve the foregoing problems, the present invention provides an aqueous humor drainage implant for draining aqueous humor in an eye to exterior of the conjunctiva for glaucoma treatment, the aqueous humor drainage implant including: a guiding tube part for guiding the aqueous humor to exterior of the eye; and a filter part, connected to one end of the guiding tube part, for preventing reflux infection from the exterior to interior of the eye, wherein the guiding tube part includes an eye-side guiding part and an outside-conjunctiva guiding tube part.

According to the invention, in installing the aqueous humor drainage implant for glaucoma treatment (simply "aqueous humor drainage implant" hereinafter) in the patient, the guiding tube part can easily be positioned with reduced invasiveness based on the anatomical structure of the eye and nearby organs. That is, in the aqueous humor drainage implant of the present invention, because the guiding tube part for guiding the aqueous humor in the eye to the filter part externally positioned to the eye has the eye-side guiding tube part and the outside-conjunctiva guiding tube part, the shape and characteristics of each tube part can be independently determined depending on the intended position where the tube is placed.

Specifically, moderate flexibility and good biocompatibility are required for the eye-side guiding tube part positioned in the living tissues such as in the anterior chamber or sclera, or under the conjunctiva, because the eye-side guiding tube part is little affected by the eye movement. On the other hand, the outside-conjunctiva guiding tube part requires a highly flexible and highly biocompatible material because it is more directly affected by the eye movement and needs to accommodate the complex anatomical structures outside the eye.

The guiding tube part of the present invention includes the eye-side guiding tube part and the outside-conjunctiva guiding tube part. Thus, the shape and characteristics of each tube part can easily be determined as desired. Further, with the junction of the eye-side guiding tube part and the outside-conjunctiva guiding tube part positioned in the vicinity of a surface of the conjunctiva, the guiding tube part can be positioned with reduced invasiveness. Further, by tailoring the shape and structure of the outside-conjunctiva guiding tube part for individual patients, damage to the eye, nasolacrimal duct, or other organs can be avoided after the aqueous humor drainage implant is installed.

Further, with the filter part connected to one end (outside-conjunctiva stamp) of the guiding tube part of the aqueous humor drainage implant, reflux infection from the exterior to interior of the eye can be prevented. That is, the aqueous humor can be safely drained out of the eye into the exterior of the conjunctiva.

In order to solve the foregoing problems, the aqueous humor drainage implant of the present invention may be adapted so that the outside-conjunctiva guiding tube part has an outer diameter smaller than an inner diameter of the nasolacrimal duct.

According to the invention, with the guiding tube positioned in the lacrimal passage including the lacrimal canaliculus, lacrimal sac, and nasolacrimal duct, the aqueous humor can drain into the nasal cavity through the lacrimal passage. That is, the guiding tube can be positioned with reduced invasiveness. Specifically, by setting the outer diameter of the guiding tube smaller than the inner diameter of the lacrimal canaliculus where the inner diameter is the smallest in the lacrimal passage, the guiding tube can be positioned anywhere in the lacrimal canaliculus, lacrimal sac, or nasolacrimal duct without undergoing the surgical operation of, for example, making an incision to position the outside-conjunctiva guiding tube. As a result, the aqueous humor can easily drain into the nasal cavity.

The inner diameter of the lacrimal canaliculus generally ranges from about 1 mm to 1.5 mm, though there are individual differences. Thus, the outer diameter of the outside-conjunctiva guiding tube part can be made smaller than the inner diameter of the lacrimal canaliculus by confining it within the range of 0.5 mm to 1.5 mm. The filter part is shaped according to the position where it is placed, but is preferably cylindrical with an outer diameter smaller than the lacrimal canaliculus as with the outside-conjunctiva guiding tube part.

In order to solve the foregoing problems, the aqueous humor drainage implant of the present invention may be adapted so that the outside-conjunctiva guiding tube part and the filter part are shaped to have a curved outer surface and sized to have substantially the same outer diameter.

According to the invention, the outside-conjunctiva guiding tube part and the filter part can be easily positioned along the eye wall. Further, damage to the conjunctiva or the sense of foreign object can be relieved. That is, with the curved outer surface structure, the outside-conjunctiva guiding tube part and the filter part can be positioned on the conjunctiva with reduced invasiveness.

An example of such a curved outer surface structure of the outside-conjunctiva guiding tube part and the filter part is a cylinder with substantially the same outer diameter, i.e., a single tube structure connecting the outside-conjunctiva guiding tube part and the filter part.

Further, with the outside-conjunctiva guiding tube part of the invention having a smaller outer diameter than the inner diameter of the lacrimal canaliculus, the aqueous humor drainage implant and the drainage passage of aqueous humor can be suitably positioned according to patient conditions.

The outside-conjunctiva guiding tube and filter part of the aqueous humor drainage implant of the present invention can be realized by directly fitting two tubes made of soft polymer material, or by joining the two via a joint. As used herein, "substantially the same outer diameter" refers to an outer diameter that enables the tubes to be smoothly fitted, or joined via a joint.

As such, "substantially the same outer diameter" includes variations due to the flexural modulus, outer diameter-to-inner diameter ratio, or other factors associated with the soft polymer materials of the outside-conjunctiva guiding tube and the filter part. Specifically, by "substantially the same outer diameter," it means that the outer diameter of one of the outside-conjunctiva guiding tube and the filter part is no greater than two times, or more preferably no greater than 1.5 times the outer diameter of the other.

In order to solve the foregoing problems, the aqueous humor drainage implant of the present invention may be adapted so that the filter part includes a hollow fiber membrane made of at least one kind of polymer material selected from the group consisting of a polyolefin polymer, a polyvinyl alcohol polymer, an ethylene-vinyl alcohol copolymer, a polysulfone polymer, a polyacrylonitrile polymer, a cellulose polymer, cellulose acetate polymer, a polymethyl methacrylate polymer, and a polyamide polymer.

According to the invention, the filter part can easily prevent reflux infection at the viral level. That is, with the extremely small pore diameter, the hollow fiber membrane can prevent reflux infection at the viral level.

Further, it is preferable that the hollow fiber membrane have an average pore diameter of no greater than 0.3 µm, or more preferably no greater than 0.02 µm. In this way, the hollow fiber membrane can reliably capture viral particles. As used herein, the "average pore diameter" of the hollow fiber membrane is a converted value obtained by a method commonly used for hollow fiber membranes for artificial kidneys, as described in Takeshi SATO et al., Functions and Adaptations of Various Blood Purification Methods-Performance Evaluation and Functional Classification of Blood Purifier, The Journal of Japanese Society for Dialysis Therapy, 29(8), 1231-1245, 1996, Japanese Society for Dialysis Therapy.

In order to solve the foregoing problems, the aqueous humor drainage implant of the present invention may be adapted so that the filter part includes a chemically bound anionic group or cationic group.

According to the invention, the reflux infection at the viral level can be prevented more reliably, and a sufficient amount of aqueous humor can be drained to reduce intraocular pressure. With the ability to electrically block viruses, the filter part can block viruses more effectively as compared with a filter part without such ability. That is, given the same pore diameter, the hollow fiber membrane can block viruses more effectively when it has a chemically bound anionic group or cationic group given by the electrical treatment. This enables the pore diameter of the hollow fiber membrane to be increased while maintaining desirable virus blocking ability, thereby readily draining aqueous humor in a sufficient amount to reduce intraocular pressure.

In order to solve the foregoing problems, the aqueous humor drainage implant of the present invention may be adapted so that the guiding tube part and the filter part are rendered hydrophilic.

According to the invention, the guiding tube part and the filter part can have improved biocompatibility, and the filter part can drain a sufficient amount of aqueous humor necessary to reduce intraocular pressure.

In order to solve the foregoing problems, the aqueous humor drainage implant of the present invention may be adapted to further include a joint part for detachably connecting the eye-side guiding tube part and the outside-conjunctiva guiding tube part.

According to the invention, the outside-conjunctiva guiding tube part can be detached at the joint part to replace the filter part as required. That is, the filter part can be replaced when it is deteriorated or damaged over the course of using the aqueous humor drainage implant. In this way, the intraocular pressure relieving effect of the aqueous humor drainage implant can be sustained for extended periods of time in a simpler, less expensive, and more patient friendly method, as compared with the case where the entire aqueous humor drainage implant is reinstalled.

Further, the invention is advantageous in that the aqueous humor drainage implant can shaped and positioned to accommodate individual differences of eyes and surrounding tissues of the patients to a certain extent. For example, with the eye-side guiding tube parts and outside-conjunctiva guiding tube parts prepared according to individual differences of eyes and tissues of the patients, suitable combinations of eye-side guiding tube part and outside-conjunctiva guiding tube part can be made according to the individual differences among the patients. That is, the shape and position of the eye-side guiding tube part and outside-conjunctiva guiding tube part can be readily adjusted to a certain extent as compared with the construction in which the guiding tube is provided in one piece.

In order to solve the foregoing problems, the aqueous humor drainage implant of the present invention may be adapted so that the outside-conjunctiva guiding tube part has a flexural modulus of no greater than 2000 Mpa at ordinary temperature.

In this way, the invention can effectively prevent problems caused by eye movement, such as invasiveness to the ocular tissue, patient's pain, and shifting of the aqueous humor drainage implant. That is, with the outside-conjunctiva having a flexural modulus of no greater than 2000 Mpa at ordinary temperature, the aqueous humor drainage implant can easily deform according to eye movement, and can have flexibility enough to relieve the invasiveness to the ocular tissue. By thus absorbing the influence of eye movement by the outside-conjunctiva guiding tube part in particular, the invention can more effectively prevent problems associated with the outside-conjunctiva guiding tube part, such as invasiveness to the ocular tissue, patient's pain, and shifting of the aqueous humor drainage implant.

In order to solve the foregoing problems, an aqueous humor drainage implant of the present invention may be adapted so that the outside-conjunctiva guiding tube part includes an outside-conjunctiva eye-side guiding tube part and an outside-conjunctiva filter-side guiding tube part, and that the outside-conjunctiva eye-side guiding tube part and the outside-conjunctiva filter-side guiding tube part are connected to each other, and wherein the outside-conjunctiva eye-side guiding tube part has a smaller flexural modulus than the outside-conjunctiva filter-side guiding tube part at ordinary temperature.

According to the invention, the influence of eye movement is more reliably absorbed by the outside-conjunctiva guiding tube part, which is particularly susceptible to the influence of eye movement. Thus, the invention can effectively prevent problems such as invasiveness to the ocular tissue, patient's pain, and shifting of the aqueous humor drainage implant. That is, by taking advantage of the fact that flexibility improves as the flexural modulus is decreased, the flexural modulus of the outside-conjunctiva eye-side guiding tube part at ordinary temperature is made smaller than that of the outside-conjunctiva filter-side guiding tube part at ordinary temperature. In this way, the influence of eye movement is absorbed by the outside-conjunctiva eye-side guiding tube part, the outside-conjunctiva filter-side guiding tube part and the filter part can be reliably protected from the influence of eye movement.

As used herein, the "flexural modulus" of a tube refers to a value measured and calculated at ordinary temperature by a common method (ASTM D790).

For a fuller understanding of the nature and advantages of the invention, reference should be made to the ensuing detailed description taken in conjunction with the accompanying drawings.

### BRIEF DESCRIPTION OF DRAWINGS

Fig. 1 is a schematic diagram illustrating how an aqueous humor drainage implant for the treatment of glaucoma according to one embodiment of the present invention is positioned in the eye when it is inserted into the nasolacrimal duct.
Fig. 2 is a diagram illustrating the overall structure of the aqueous humor drainage implant for the treatment of glaucoma shown in Fig. 1, separately as an anterior part and a posterior part.
Fig. 3 is a cross sectional view illustrating an example in which a plurality of pores are provided at the front end of an outer sheath part, schematizing a structure of a filter part of the aqueous humor drainage implant for the treatment of glaucoma shown in Fig. 1, cut along the direction of extension of the filter part.
Fig. 4 is a cross sectional view of the filter part taken along the line A-A', schematizing a structure of the filter part of the aqueous humor drainage implant for the treatment of glaucoma shown in Fig. 1.
Fig. 5 is a schematic diagram illustrating how the aqueous humor drainage implant for the treatment of glaucoma shown in Fig. 1 is positioned on the conjunctiva of the eye.

### BEST MODE FOR CARRYING OUT THE INVENTION

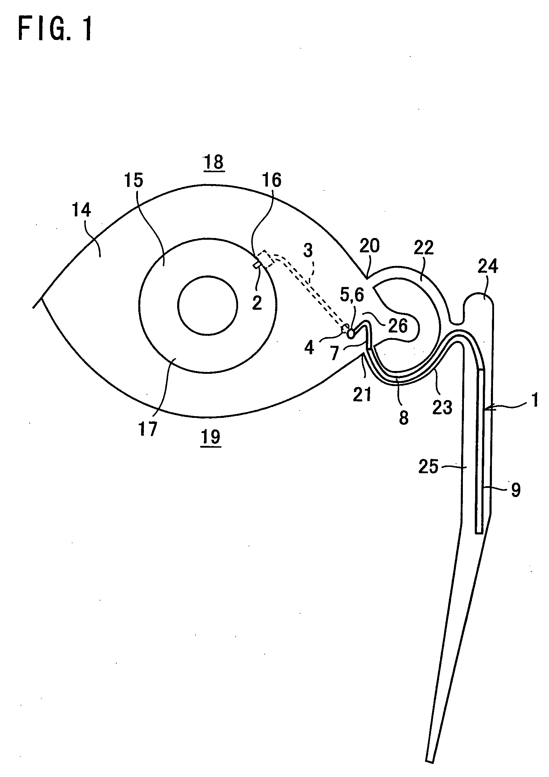

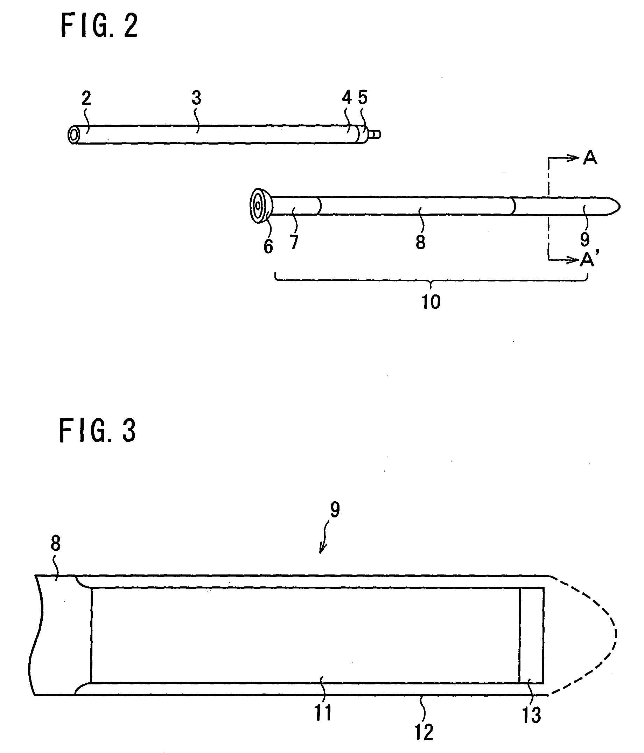

Referring to Fig. 1 through Fig. 5, the following will describe an exemplary structure of an aqueous humor drainage implant for glaucoma treatment (hereinafter simply referred to as "aqueous humor drainage implant"). Fig. 1 schematizes how an aqueous humor drainage implant according to one embodiment of the present invention is positioned in an eye by being inserted into the nasolacrimal duct. Fig. 2 illustrates the overall structure of the aqueous humor drainage implant shown in Fig. 1, separately as an anterior part and a posterior part.

As shown in Fig. 1 and Fig. 2, the aqueous humor drainage implant of the present embodiment has three major parts: a first tube (guiding tube part, eye-side guiding tube part) 3; first and second joints (joint parts) 5 and 6; and a posterior part 10. Note that, in the following, the first joint 5 and the second joint 6 will be collectively referred to simply as joints 5 and 6, unless otherwise noted.

Specifically, the first tube 3 constitutes the anterior part of the aqueous humor drainage implant 1, connecting the anterior chamber of the eye to the exterior of a conjunctiva 14, and positioned along the sclera wall under the conjunctiva 14. The posterior part 10 of the aqueous humor drainage implant 1 is positioned such that it extends from an angulus oculi medialis 26, through an upper lacrimal punctum 20 (or lower lacrimal punctum 21), into an upper lacrimal canaliculus 22 (or lower lacrimal canaliculus 23), a lacrimal sac 24, or a nasolacrimal duct 25. The joints 5 and 6 of the aqueous humor drainage implant 1 connect the first tube 3 (anterior part) to the posterior part 10. Note that, in the example shown in Fig. 1, the posterior part 10 is positioned in the lacrimal sac 24 or the nasolacrimal duct 25. Alternatively, the posterior part 10 may be positioned on the conjunctiva 14, as will be described later. As used herein, the term "conjunctiva" 14 includes the bulbar conjunctiva, conjunctiva cul-de-sac, and palpebral conjunctiva.

The posterior part 10 includes a second tube (guiding tube part, outside-conjunctiva guiding tube part, outside-conjunctiva eye-side guiding tube part) 7, a third tube (guiding tube part, outside-conjunctiva guiding tube part, outside-conjunctiva filter-side guiding tube part) 8, and a filter part 9. The first tube 3, the second tube 7, and the third tube 8 correspond to a guiding tube part of the present invention, and the filter part 9 corresponds to a filter part of the present invention. Further, the joints 5 and 6 correspond to a joint part of the present invention, and the second tube 7 and the third tube 8 correspond to an outside-conjunctiva guiding tube part of the present invention. Further, the second tube 7 and the third tube 8 correspond to an outside-conjunctiva eye-side guiding tube part and an outside-conjunctiva filter-side guiding tube part of the present invention, respectively.

With this arrangement, the aqueous humor in the anterior chamber of the eye is guided into the joints 5 and 6 through the first tube 3. Through the joints 5 and 6, the aqueous humor is ejected out of the conjunctiva 14 and, via the posterior part 10, drains into the upper lacrimal canaliculus 22 (or lower lacrimal canaliculus 23), the lacrimal sac 24, or the nasolacrimal duct 25. The aqueous humor drained out of the aqueous humor drainage implant 1 flows through the upper lacrimal canaliculus 22 (or lower lacrimal canaliculus 23) and the lacrimal sac 24, and is absorbed in the nasolacrimal duct 25 and the nasal cavity (not shown) connecting to the nasolacrimal duct 25.

Although Fig. 1 illustrates an example in which the aqueous humor flown through the posterior part 10 is absorbed in the nasal cavity, the aqueous humor may alternatively be drained onto the conjunctiva 14 through the posterior part 10, as will be described later. In this case, the aqueous humor is absorbed by the tissues of the conjunctiva 14.

To describe the first tube 3 of the aqueous humor drainage implant 1 of the present embodiment more specifically, the first tube 3 constituting the anterior part is a single silicon tube with an inner diameter of 0.5 mm, an outer diameter of 1.0 mm, and a length of 10 mm, and is connected to the joint 5 at a conjunctiva-side stump 4. The first tube 3 is surgically positioned along the sclera wall under the conjunctiva 14. Here, an anterior chamber-side stump 2 of the first tube 3 is inserted into the anterior chamber of the eye, and the conjunctiva-side stump 4 and the joint 5 are placed external of the conjunctiva 14 at the angulus oculi medialis 26.

Specifically, a flap of conjunctiva 14 and the underlying tissue is opened with an incision to expose a sclera 16. Here, any breeding should be controlled. The conjunctiva-side stump 4 of the first tube 3 of the aqueous humor drainage implant 1 is secured with a suture to the sclera wall at the angulus oculi medialis 26. Then, the anterior chamber-side stump 2 of the first tube 3 of the aqueous humor drainage implant 1 is inserted into the sclera 16 by a known method, and positioned in the anterior chamber by inserting it between an iris 17 and a cornea 15.

The first tube 3 is properly secured to the sclera wall with a suture, so as to position it as shown in Fig. 1. The conjunctiva 14 is then restored and the incision is closed with a suture. Here, the incision of conjunctiva 14 around the conjunctiva-side stump 4 of the first tube 3 is closed using a known method employing, for example a purse-string suture, a biologically acceptable adhesive, and the like, so that the first joint 5 connected to the conjunctiva-side stump 4 of the first tube 3 is exposed external of the conjunctiva 14.

With the first tube 3 and the joint 5 positioned in the described manner, the aqueous humor in the anterior chamber can be guided into the exterior of the conjunctiva 14 through the first tube 3 and the joint 5.

Considering that the first tube 3 is positioned in the living tissues such as the anterior chamber, the conjunctiva 14, and the sclera 16, any material can be used for the first tube 3 as long as it is sufficiently flexible and has good biocompatibility. Specific examples of the first tube 3 are various polymers, including: silicone resins; polyolefin resins such as polyethylene, polypropylene, polyisobutylene, ethylene-vinyl acetate copolymer, and polynorbornene; polyurethane resins; synthetic rubbers such as polybutadiene, polyisoprene, SBR (Styrene Butadiene Rubber), and SIR; and natural rubbers. In light of the proved performance and reliability, silicone resins and polyurethane resins are preferably used.

It is preferable that the first tube 3 have substantially the same outer diameter as the posterior part 10 which the first tube 3 is connected to. Generally, the outer diameter of the first tube 3 ranges from about 0.5 mm to about 1.5 mm, ignoring individual differences. Namely, a suitable outer diameter of the first tube 3 generally ranges from about 0.5 mm to about 1.5 mm, though it varies from patient to patient requiring the aqueous humor drainage implant 1. Further, the first tube 3 is generally about 5 mm to 20 mm in length, though it depends on where the anterior chamber-side stump 2 is inserted.

As described above, the first tube 3 is secured along the sclera wall. To this end, the first tube 3 may have any structure using known techniques, so long as it assists the procedure of securing the first tube 3. For example, using a known technique, the first tube 3 may have a projection-like structure along its outer surface. With such a structure, the first tube 3 can be secured along the sclera wall more easily.

The conjunctiva-side stump 4 of the first tube 3 is connected to the first joint 5. For this purpose, the conjunctiva-side stump 4 and the first joint 5 are desirably fastened together in advance. In this way, surgical procedures can be performed more easily, and infection from the junction can be prevented more reliably.

As illustrated in Fig. 1 and Fig. 2, the posterior part 10 of the aqueous humor drainage implant 1 of the present embodiment has three parts, including: the second tube 7 connected to the second joint 6; the filter part 9; and the third tube 8 bridging the second tube 7 and the filter part 9. With the shape and structure described below, the posterior part 10 can be positioned with reduced invasiveness in any of the upper lacrimal canaliculus 22, the lower lacrimal canaliculus 23, the lacrimal sac 24, the nasolacrimal duct 25, or on the conjunctiva 14, an effect which has not been realized with conventional aqueous humor drainage implants.

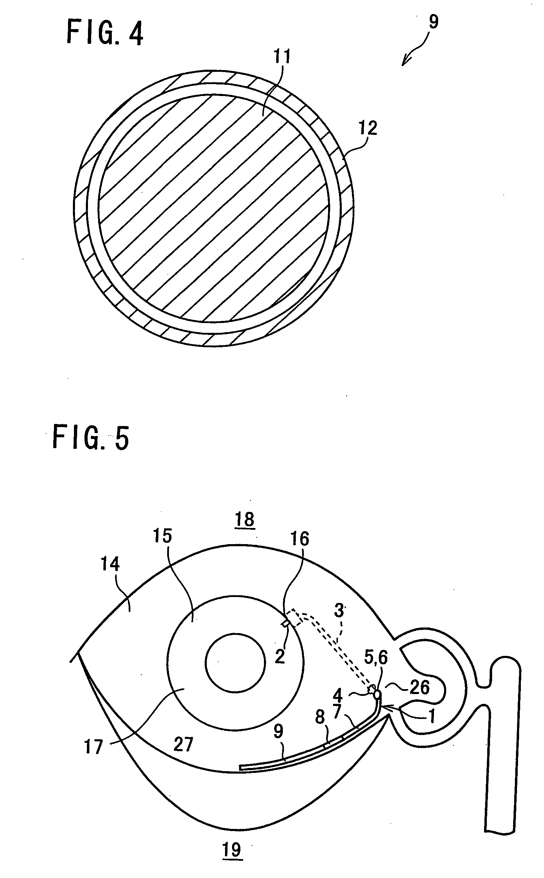

First, description is made below as to the posterior part 10 positioned in the upper lacrimal canaliculus 22, the lower lacrimal canaliculus 23, the lacrimal sac 24, or the nasolacrimal duct 25, as shown in Fig. 1. In the human body, there is a canaliculus, called the lacrimal duct, that passes the lacrimal fluid from the angulus oculi medialis 26 to the nasal cavity (not shown), as illustrated in Fig. 1 and Fig. 5. The lacrimal duct is a single duct with a diameter of about 1 mm to 1.5 mm, and a length of 10 mm to 30 mm. The lacrimal duct includes the upper lacrimal punctum 20, the lower lacrimal punctum 21, the upper lacrimal canaliculus 22, the lower lacrimal canaliculus 23, the lacrimal sac 24, and the nasolacrimal duct 25, and connects the angulus oculi medialis 26 to the nasal cavity. (The lacrimal duct communicates between the angulus oculi medialis 26 and the nasal cavity.)

The present invention enables the posterior part 10 to be inserted into the lacrimal duct by taking advantage of the anatomical feature of the lacrimal duct draining the lacrimal fluid into the nasal cavity. That is, the aqueous humor drainage implant 1 of the present embodiment enables the aqueous humor in the anterior chamber to be drained into the nasal cavity with the structure of the lacrimal duct intact, thereby realizing installation with reduced invasiveness, unattained by conventional techniques. Namely, with the posterior part 10 shaped into a single tube to be inserted into the lacrimal duct as described below, the aqueous humor drainage implant 1 of the present embodiment can be inserted into the lacrimal duct with reduced invasiveness.

Next, the following will describe the case where the posterior part 10 is positioned on the conjunctiva 14. Fig. 5 schematizes how the aqueous humor drainage implant 1 of the present embodiment is positioned on the conjunctiva of the eye. It should be noted here that members and portions having the same functions are those described with reference to Fig. 1 are given the same reference numerals and explanations thereof are omitted.

In positioning the posterior part 10 on the conjunctiva 14 as shown in Fig. 5, care must be taken not to damage the cornea or conjunctiva with the posterior part 10 after it is placed in position, or not to cause any discomfort to the patient, in addition to avoiding any invasiveness due to the installation. To this end, the posterior part 10 needs to be positioned on the conjunctiva 14 along the curved surface of the eye wall, as shown in Fig. 5, so as to minimize the influence of eye movement, instead of simply hanging the aqueous humor drainage implant in the conjunctival sac as in the conventional technique.

By being positioned on the conjunctiva 14 as shown in Fig. 5, the posterior part 10 becomes part of the eye through the conjunctiva 14, and is therefore able to follow the eye movement in any direction. That is, with the single tube construction, the posterior part 10 can easily be positioned along the eye wall. Further, the single tube construction allows the posterior part 10 to be positioned on the conjunctiva 14 with reduced invasiveness, without causing much damage or discomfort to the conjunctiva 14 by the posterior part 10 positioned thereon. Note that, in the example shown in Fig. 5, the posterior part 10 is positioned on the conjunctival cul-de-sac 27 of the conjunctiva 14.

It should be noted here that the posterior part 10 is connected to the second joint 6 regardless of whether the posterior part 10 is positioned in the lacrimal duct as shown in Fig. 1, or on the conjunctiva 14 along the eye wall as shown in Fig. 5. For this reason, the posterior part 10 is directly influenced by the eye movement. However, the influence of eye movement is minimized by the segmented structure of the posterior part 10 divided into the second tube 7, the third tube 8, and the filter part 9.

That is, by setting required levels of flexibility and biocompatibility for each of these different parts, the influence of eye movement on the posterior part 10 can be minimized. In the present embodiment, the posterior part 10 is segmented into three parts; however, the number of segments is not just limited to three.

As described above, the posterior part 10 has a single tube structure, and is segmented into plural parts, specifically, the second tube 7, the third tube 8, and the filter part 9. This enables the posterior part 10 of the aqueous humor drainage implant 1 to be positioned with reduced invasiveness in any of the upper lacrimal canaliculus 22, the lower lacrimal canaliculus 23, the lacrimal sac 24, and the nasolacrimal duct 25, or on the conjunctiva 14. Details of the single tube structure of the posterior part 10 will be described later.

As described above, the posterior part 10 desirably has substantially the same outer diameter as the first tube 3, preferably and generally in a range of 0.5 mm to 1.5 mm.

More specifically, in the aqueous humor drainage implant 1 of the present embodiment, the second tube 7 and the third tube 3 are both silicon tubes with an inner diameter of 0.5 mm and an outer diameter of 1.0 mm. That is, the posterior part 10 includes two silicon tubes. The second tube 7 is 5 mm in length, and the third tube 8 is 10 mm to 30 mm in length. The filter part 9 is constructed from, for example, a polyethylene tube sheath with an inner diameter of 0.8 mm, an outer diameter of 1.0 mm, and a length of 10 mm, and an 8 mm-long hollow fiber membrane provided therein with an outer diameter of 0.7 mm.

Using the hollow fiber membrane as a filter enables the filter part 9 to be constructed as a single tube like the second tube 7 and the third tube 8. The second tube 7, the third tube 8, and the filter part 9 are shaped and sized based on the anatomy of the lacrimal duct, so as to enable the posterior part 10 to be positioned with reduced invasiveness in any of the upper lacrimal canaliculus 22, the lower lacrimal canaliculus 23, the lacrimal sac 24, and the nasolacrimal duct 25. Further, the anatomy of the eyeball is also taken into account in designing the shape and size of the second tube 7, the third tube 8, and the filter part 9, so that the posterior part 10 can be positioned on the conjunctiva 14 with reduced invasiveness.

As described above, depending on patient conditions, the posterior part 10 is surgically positioned in any of the upper lacrimal canaliculus 22, the lower lacrimal canaliculus 23, the lacrimal sac 24, and the nasolacrimal duct 25, or on the conjunctiva 14. In the case where the posterior part 10 is positioned in the upper lacrimal canaliculus 22, the lower lacrimal canaliculus 23, the lacrimal sac 24, or the nasolacrimal duct 25 as shown in Fig. 1, the posterior part 10 is inserted into the upper lacrimal punctum 20 (or lower lacrimal punctum 21) by a known nasolacrimal duct bougienage method, and is positioned in the upper lacrimal canaliculus 22, (lower lacrimal canaliculus 23), the lacrimal sac 24, or the nasolacrimal duct 25.

Here, the second tube 7 is positioned outside the upper lacrimal punctum 20 (or lower lacrimal punctum 21) so as to allow the posterior part 10 to follow the eye movement. The stump of the second tube 7 is connected to the first joint 5 via the second joint 6. In the case where the posterior part 10 is positioned on the conjunctiva 14 as shown in Fig. 5, the stump of the second tube 7 is connected to the first joint 5 via the second joint 6, and the posterior part 10 is positioned on the conjunctiva 14. Note that, in Fig. 1 and Fig. 5, the upper eyelid and lower eyelid are shown as 18 and 19, respectively.

As used herein, "patient conditions" refers to situations where the nasolacrimal duct is clogged, the drained aqueous humor affects vision, or the patient feels discomfort by the presence of the posterior part 10. For example, for patients suffering from a clogged nasolacrimal duct, the posterior part 10 is desirably positioned on the conjunctiva 14. If, for example, the posterior part 10 positioned on the conjunctiva 14 leads to affected vision by the drained aqueous humor, or discomfort (unpleasant sensation) due to the posterior part 10 during eye movement, the posterior part 10 is desirably positioned in the upper lacrimal canaliculus 22, the lower lacrimal canaliculus 23, the lacrimal sac 24, or the nasolacrimal duct 25.

In any case, the lengths of the second tube 7 and third tube 8 can be adjusted as required to accommodate different patient conditions. Further, the second joint 6 and the posterior part 10 are desirably fastened together in advance. In this way, surgical procedures can be performed more easily, and infection from the junction of the second joint 6 and the posterior part 10 can be prevented more reliably.

Here, because the first tube 3 and the joints 5 and 6 are secured to the sclera wall, the second tube 7 extending therefrom is under the direct mechanical force of eye movement. Here, eye movement is restricted if the second tube 7 is not elastic enough to follow the eye movement within the movable range of the eye. This may lead to ambiopia or displacement of the aqueous humor drainage implant 1.

In order to prevent ambiopia or displacement of the aqueous humor drainage implant 1, the second tube 7 particularly requires good elasticity, flexibility, and ease of deformation sufficient to accommodate the eye movement. That is, it is required that the second tube 7 be made of a material that provides good elasticity, flexibility, and ease of deformation. Depending on the movable range of the eye, there are cases where the second tube 7 is brought into contact with the cornea or other ocular tissues for a brief moment. Thus, in order to ensure that the second tube 7 does not damage the ocular tissues, it is important that the second tube 7 be made of a material that offers good elasticity, flexibility, and ease of deformation. That is, the second tube 7 requires a highly flexible and biocompatible material that can easily deform to follow eye movement and that can relieve invasiveness to the ocular tissues. With the posterior part 10 including the second tube 7 satisfying such conditions, problems associated with the eye movement, such as invasiveness to the ocular tissues, pain, and displacement of the aqueous humor drainage implant 1 can be effectively prevented.

The material of the second tube 7 is not particularly limited as long as it offers good elasticity, flexibility, ease of deformation, and biocompatibility. Some of the representative examples are various types of polymer materials, including: silicone resins; polyolefin resins such as polyethylene, polypropylene, polyisobutylene, ethylene-vinyl acetate copolymer, and polynorbornene; polyurethane resins; natural rubbers; and synthetic rubbers. Among these materials, silicone resins and polyurethane resins are particularly preferable. The second tube 7 desirably has substantially the same outer diameter as the first tube 3 and the third tube 8. Further, taking into account the expansion and contraction due to the eye movement, the second tube 7 is generally about 5 mm to 20 mm in length, though it depends on where the joints 5 and 6, and the posterior part 10 are positioned. Further, the second joint 6 and the second tube 7 are desirably fastened together in advance. In this way, surgical procedures can be performed more easily, and infection from the junction can be prevented more reliably.

Considering that the third tube 8 is positioned on the conjunctiva 14, or in other living tissues such as the upper lacrimal punctum 20, the lower lacrimal punctum 21, the upper lacrimal canaliculus 22, the lower lacrimal canaliculus 23, and the lacrimal sac 24, any material can be used for the third tube 8 as long as it is sufficiently flexible and has good biocompatibility. Some of the representative examples of the third tube 8 are various polymers, including: silicone resins; polyolefin resins such as polyethylene, polypropylene, polyisobutylene, and ethylene-vinyl acetate copolymer; polyurethane resins; synthetic rubbers; and natural rubbers. Among these materials, silicone resins and polyurethane resins are particularly preferable.

Considering that the third tube 8 is positioned in the upper lacrimal canaliculus 22, the lower lacrimal canaliculus 23, the lacrimal sac 24, and the nasolacrimal duct 25, it is required that the third tube 8 have a narrower outer diameter than the inner diameter of any of the upper lacrimal punctum 20, the lower lacrimal punctum 21, the upper lacrimal canaliculus 22, the lower lacrimal canaliculus 23, and the lacrimal sac 24. Generally, the outer diameter of the third tube 8 desirably ranges from about 0.5 mm to about 1.5 mm, ignoring individual differences. Further, the third tube 8 is generally about 5 mm to 20 mm in length, though it depends on where the posterior part 10 is positioned and ignoring individual differences among patients.

The second tube 7 and the third tube 8 are highly flexible with a flexural modulus of no greater than 2000 Mpa at ordinary temperature, thereby preventing problems associated with eye movement, such as invasiveness to the eye, pain, and displacement of the aqueous humor drainage implant 1. In the present embodiment, the second tube 7 and the third tube 8 have the same flexural modulus at ordinary temperature, i.e., the same flexibility. However, the second tube 7 may have a smaller flexural modulus than the third tube 8 at ordinary temperature. This enables the second tube 7 to absorb the influence of eye movement more reliably.

Depending on the elasticity of the second tube 7 or movement of the filter part 9 in the nasolacrimal duct 5, there are cases where the position of the third tube 8 may be affected. For example, with the posterior part 10 positioned in the upper lacrimal canaliculus 22, the lower lacrimal canaliculus 23, the lacrimal sac 24, or the nasolacrimal duct 25, the third tube 8 may slip out of the upper lacrimal punctum 20 or the lower lacrimal punctum 21, or drawn into the nasolacrimal duct 25.

Such situations can be avoided by securing the posterior part 10 to a suitable position. The method of securing the posterior part 10 is not particularly limited, and conventional methods can be used. For example, the following methods can be used when the posterior part 10 is positioned in the upper lacrimal canaliculus 22, the lower lacrimal canaliculus 23, the lacrimal sac 24, or the nasolacrimal duct 25. In the first method, the upper lacrimal punctum 20 or the lower lacrimal punctum 21 is tightened by ligation. In the second method, the second tube 7 or the third tube 8 is temporarily secured to the skin around the upper lacrimal punctum 20 or the lower lacrimal punctum 21 by ligation. In the third method, a wing-like projection serving as a stopper is provided at the boundary of the second tube 7 and the third tube 8.

In the case where the posterior part 10 is positioned on the conjunctiva 14, the third tube 8 may be omitted as required to directly join the second tube 7 and the filter part 9. However, regardless of whether the third tube 8 is omitted or not, there is always a possibility, whenever the posterior part 10 is positioned on the conjunctiva 14, that the posterior part 10 moves out of position and becomes unstable due to the eye movement. This can be avoided by securing the posterior part 10 to a suitable position on the conjunctiva 14. The method of securing the posterior part 10 on the conjunctiva 14 is not particularly limited, and conventional methods can be used. As one example, the posterior part 10 may be secured to the conjunctiva 14 by a suture.

Fig. 3 is a cross sectional view schematizing a structure of the filter part cut along the direction of extension of the filter part 9 of the aqueous humor drainage implant 1 of the present embodiment. As shown in Fig. 3, the filter part 9 includes a hollow fiber membrane part 11 and an outer sheath part 12. It should be noted here that the outer sheath part 12 is optionally provided according to the hardness of the hollow fiber membrane part 11. As such, the filter part 9 may only include the hollow fiber membrane part 11.

Fig. 4 is a cross sectional view of the filter part taken along the line A-A', schematizing a structure of the filter part of the aqueous humor drainage implant shown in Fig. 1. As illustrated in Fig. 4, the filter part 9 of the present embodiment includes the hollow fiber membrane part 11 inside the outer sheath section 12.

Considering that the filter part 9 is positioned in the upper lacrimal canaliculus 22, the lower lacrimal canaliculus 23, the lacrimal sac 24, and the nasolacrimal duct 25, it is required that the filter part 9 have a narrower outer diameter than the inner diameter of any of the upper lacrimal punctum 20, the lower lacrimal punctum 21, the upper lacrimal canaliculus 22, the lower lacrimal canaliculus 23, and the lacrimal sac 24. Generally, the outer diameter of the filter part 9 desirably ranges from about 0.5 mm to about 1.5 mm, ignoring individual differences among patients. As such, the outer diameter of the filter part 9 is desirably about 0.5 mm to 1.5 mm. Further, the filter part 9 is generally about 5 mm to 20 mm in length, though it depends on individual differences among patients. Note that, if the filter part 9 includes only the hollow fiber membrane part 11, the outer diameter of the filter part 9 coincides with the outer diameter of the hollow fiber membrane part 11. On the other hand, if the filter part 9 includes the outer sheath part 12, the outer diameter of the filter part 9 coincides with the outer diameter of the outer sheath part 12.

As shown in Fig. 3, the hollow fiber membrane part 11 opens into the third tube 3 at an end connecting thereto, and is closed at a stump 13 to provide a dead end. That is, the aqueous humor that flows into the aqueous humor drainage implant 1 from the anterior chamber of the eye all passes through the hollow fiber membrane part 11 and drains out of the filter part 9 through pores formed through a side wall of the hollow fiber membrane part 11.

As to a method of closing the stump 13 of the hollow fiber membrane part 11, any conventional method can be used as long as it can close the stump 13. For example, a method using a polyurethane adhesive, or a method employing heat fusion is available.

The hollow fiber membrane part 11 of the filter part 9 is provided to reduce intraocular pressure by draining the aqueous humor, and to prevent viruses, bacteria, fungi, or other microorganisms that exist outside the conjunctiva 14 from entering the first tube 3 and the posterior part 10. In this way, intraocular pressure is reduced, and at the same time, reflux infection from the conjunctiva 14 is prevented.

For the purpose of draining aqueous humor, the hollow fiber membrane 11 must accommodate an aqueous humor production rate in a range of 2.0 µl/min to 3.0 µl/min, and attain a target intraocular pressure of about 10.0 mmHg to 20.0 mmHg. As such, the hollow fiber membrane part 11 must provide an aqueous humor flow rate of no less than 2.0 µl/min to 3.0 µl/min under a hydraulic pressure of about 10.0 mmHg to 20.0 mmHg.

In order to examine whether a hollow fiber membrane used for the hollow fiber membrane part 11 of the aqueous humor drainage implant 1 of the present embodiment satisfy these conditions, following series of experiments were conducted.

### [Experimental method for the evaluation of hollow fiber membrane]

With a hollow fiber membrane used for the hollow fiber membrane part 11 of the aqueous humor drainage implant 1 of the present embodiment, the amount of aqueous humor flown under a certain hydraulic pressure was measured. Specifically, in a vertically placed pipe, the pseudo aqueous humor "BSS plus" (the product of SANTEN PHERMACEUTICAL CO., LTD.) was charged to about 13 cm from the bottom end of the pipe. Then, with the hollow fiber membrane part 11, 10 mm long, fitted to the bottom end of the pipe, the outflow weight of BSS plus per unit time was measured. From the measured weight of BSS plus and its specific gravity, an outflow volume of BSS plus was calculated. As the hollow fiber membrane used as the hollow fiber membrane part 11, two kinds of prototype EVAL membranes with different average pore diameters and different outer diameters were used. Note that, the foregoing procedure was carried out with the BSS plus maintained at 37°C.

### [Preparation method of prototype EVAL membranes]

For the preparation of the prototype EVAL membranes, a starting solution was prepared first by heating, stirring, and dissolving at 90°C 15 parts by weight of ethylene-vinyl alcohol copolymer with the ethylene content of 32 mol% and saponificated to 99 mol% (the KURARAY Co., LTD. product EVAL EC-F100A), 73 parts by weight of dimethylsulfoxide, 10 parts by weight of water, and 2 parts by weight of lithium chloride.

The starting solution so prepared had a LST (Lower Solution Temperature) of 28°C. The starting solution was a transparent homogeneous solution at high temperatures, but underwent phase separation and became clouded with decreasing temperature. When allowed to stand for extended periods of time, the solution separated into two layers. In the present embodiment, a temperature at which such phase separation occurs will be referred to as the LST.

Using a double annular nozzle, the starting solution maintained at 40°C was extruded with water injected through the center of the nozzle. Then, the solution was allowed to pass through an air layer and was solidified in a water bath. After water washing, wet heating, drying, and heat treatment according to ordinary method, a dry hollow fiber membrane was obtained as a hollow fiber membrane E1, i.e., the prototype EVAL membrane.

In addition, another kind of prototype EVAL membrane, hollow fiber membrane E2, was prepared under the same conditions as for the hollow fiber membrane E1, except that the ethylene-vinyl alcohol copolymer saponificated to 99 mol% was used in 17 parts by weight, and that the dimethylsulfoxide was used in 71 parts by weight. The starting solution used for the preparation of the hollow fiber membrane E2 had a LST of 30°C.

The preparation method of the prototype EVAL membrane, i.e., the ethylene vinyl alcohol copolymer is described in more detail in Japanese Laid-Open Patent Publication No. 286740/2001 (*Tokukaihei* 13-286740).

Table 1 below shows results of evaluation experiment for the average pore diameter and outer diameter of the hollow fiber membranes E1 and E2. As shown in Table 1, the hollow fiber membranes E1 and E2 both had a flow rate that satisfied the required conditions for the production rate of aqueous humor noted above. The results therefore showed the effectiveness of the aqueous humor drainage implant 1 of the present invention in draining the aqueous humor and thereby keeping the intraocular pressure within a normal range.

**[Table 1]**

| Hollow fiber membrane | Average pore size (µm) | Outer diameter of hollow fiber membrane (µm) | The number of hollow fiber membranes | Flow rate of pseudo aqueous humor (µl/min) |
|---|---|---|---|---|
| E1 | 0.004 | 780 | 1 | 5.68 |
| E2 | 0.005 | 300 | 4 | 2.2 |

It should be noted here that, depending on the performance of the hollow fiber membrane 11, there are cases where the aqueous humor drainage implant 1 of the present invention may drain the aqueous humor in excess. Such excess draining of the aqueous humor may lead to low intraocular pressure after the surgical operation. In order to prevent such a situation, a pressure-controlled check valve or regulator valve may be suitably provided in the first tube 3, the first joint 5, the second joint 6, or the posterior part 10 according to the performance of the hollow fiber membrane part 11 used.

The pressure-controlled check valve opens and closes to maintain the intraocular pressure within the normal intraocular pressure range of about 7 mmHg to 20 mmHg. Any type of conventional pressure-controlled check valve may be used as long as it has a structure meeting this purpose. For example, a slit check valve used for Krupin-Denver eye shunt (USP 5,454,796) and a check valve used for the Ahmedglaucoma implant (USP 5,071,408, USP 6,261,256) may be used. The pressure-controlled check valve, with its check valve structure, prevents backflow of the aqueous humor in situations where there is abrupt pressure increase inside the nasolacrimal duct as in nose blowing or sneezing.

From the standpoint of preventing reflux infection due to viruses or other microorganisms, the hollow fiber membrane of the hollow fiber membrane part 11 needs to have an average pore diameter of no greater than 0.3 µm, preferably 0.0001 µm to 0.02 µm, or more preferably 0.0001 µm to 0.01 µm, taking into account the diameter of viral particles ranging from about 0.02 µm to 0.3 µm. With ah average pore diameter of hollow fiber membrane exceeding these ranges, it may become increasingly difficult to block viral particles.

However, the foregoing condition required for the average pore diameter of the hollow fiber membrane is adjustable within a range that can achieve the object of the hollow fiber membrane 11, i.e., to prevent reflux infection at the viral level. To describe more specifically, in the case where the hollow fiber membrane 11 has the additional function of electrically blocking viruses as will be described later, the viruses are also captured electrically, in addition to being captured by the small average pore diameter of the hollow fiber membrane. That is, the hollow fiber membrane used for the hollow fiber membrane part 11 may have an average pore diameter greater than the foregoing ranges as long as it serves to prevent reflux infection at the viral level.

The material of the hollow fiber membrane used for the hollow fiber membrane part 11 is not particularly limited as long as it is moderately water permeable and serves to prevent reflux infection at the viral level. For example, various polymers such as a polyolefin polymer, a polyvinyl alcohol polymer, an ethylene-vinyl alcohol copolymer, a polysulfone polymer, a polyacrylonitrile polymer, a cellulose polymer, cellulose acetate polymer, a polymethyl methacrylate polymer, and a polyamide polymer are available.

Applicable areas of hollow fiber membrane extend to various fields. In medical applications, the hollow fiber membrane has been used primarily in artificial kidneys. Generally, the hollow fiber membrane used for this purpose has an average pore diameter of about 0.005 µm to 0.008 µm, and this satisfies the foregoing condition required for the hollow fiber membrane of the hollow fiber membrane part 11 of the aqueous humor drainage implant 1 of the present invention. Thus, the hollow fiber membrane for the present invention can be suitably selected from industrially available hollow fiber membranes for artificial kidneys.

Specific examples of such a hollow fiber membrane for artificial kidneys include those used for the dialyzer of devices such as the APS-150, AM-FP-130, AM-GP-13, AM-UP-13 (products of Asahi Kasei Medical Co., Ltd.), Meltrax 140, Meltrax 160 (products of MERA), FB-130U (product of NIPRO CORPORATION), BS-1.6 (Toray Industries, Inc.), and PS-1.6N (KAWASUMI LABORATORIES, INC.). (Seisuke TAKASHIMA, Essential Properties of Membrane Materials, Clinical Engineering, 1997, Vol. 8, No. 6, pp. 479-492).

For the purpose of preventing reflux infection at the viral level more reliably while maintaining sufficient flow rate for the aqueous humor, the hollow fiber membrane part 11 may have the function of electrically blocking viruses, in addition to capturing viruses by the pore diameter of the hollow fiber membrane.

It is known that viral particles as a whole are negatively charged under normal neutral pH range conditions as are many microorganisms. By taking advantage of this fact, passage of viral particles through the hollow fiber membrane can be prevented by negatively charging the hollow fiber membrane part 11 with chemically bound (introduced) anionic groups and thereby causing the viral particles to repel the negative ions that exist in the hollow fiber membrane. Alternatively, the hollow fiber membrane 11 may be positively charged by chemically binding cationic groups thereto. In this case, the viral particles are drawn to the hollow fiber membrane part 11 by being attracted to the positive ions that exist in the hollow fiber membrane, with the result that passage of the viral particles is prevented.

Meanwhile, the protein, which is one of the constituents of the virus, is an ampholyte, including cationic groups (primarily amino groups) and anionic groups (primarily carboxyl groups). It is envisaged that, by the same mechanism as the ion exchange membrane, the anionic group or cationic group chemically bound to the hollow fiber membrane part 11 captures the amino group or carboxyl group of the protein by forming an ion pair.

That is, by "electrically blocking viruses," it means that passage of viral particles through the hollow fiber membrane is prevented by the electric force. Further, with the ability to electrically block viruses, the hollow fiber membrane part 11 can block passage of viruses with a larger pore diameter (average pore diameter) as compared with a non-charged membrane with no electrical capabilities.

The method of introducing ionic groups into the hollow fiber membrane part 11 is not particularly limited as long as it can introduce ionic groups into the hollow fiber membrane of the hollow fiber membrane part 11. For example, methods employing known acid treatment, alkali treatment, oxidation process, photo irradiation, addition reaction, or graft reaction may be used. In the case of a polymer material having hydroxyl groups in its molecules for example, a sulfuric acid group, carboxyl group, amino group, or other ionic groups can be easily introduced by, for example, esterification, etherification, or Michael addition. (See Seisuke TAKASHIMA et al., Research on removal of HB antigen by absorbent, The Journal of Japanese Medical Instruments, 1986, vol. 56, No. 11, pp. 499-505, Japanese Patent Nos. 1695758, 1695760.)

Depending on hardness of the hollow fiber membrane part 11, the hollow fiber membrane part 11 may be optionally provided with the outer sheath part 12, in order to assist installation of the hollow fiber membrane part 11 outside the conjunctiva 14 and improve durability of the filter part 9. As illustrated in Fig. 3, the outer sheath part 12 on its front end (stump) has a plurality of pores, providing passageways for the aqueous humor drained through the sidewall of the hollow fiber membrane part 11. Note that, in the present embodiment, the outer sheath part 12 has a plurality of pores at its front end to provide passageways for the aqueous humor. However, the pores provided through the outer sheath part 12 are not limited to this arrangement as long as they can pass the aqueous humor. For example, the outer sheath part 12 may have one or more openings (pores) through the sidewall, or one or more openings (pores) through the sidewall and front end.

The material of the outer sheath part 12 is not particularly limited as long as it can provide adequate hardness and good biocompatibility. Some of the examples include various polymer materials, including silicone resin, polyethylene resin, polypropylene resin, polyvinyl alcohol resin, ethylene-vinyl alcohol copolymer, polyurethane resin, synthetic rubber, natural rubber, trans-polyisoprene resin, and polycarbonate resin. Among these materials, silicone resin, polyurethane resin, and trans-polyisoprene resin are particularly preferable.

For the purpose sustaining a flow rate of the aqueous humor in the hollow fiber membrane part 11 and improving biocompatibility of the posterior part 10, the posterior part 10 may be rendered hydrophilic. For the hydrophilic treatment, any conventional method may _be used. For example, methods employing surface grafting, oxidation process, acid treatment, alkali treatment, and Michael addition are available.

Joining the first tube 3 and the posterior part 10 with the joints 5 and 6 allows the posterior part 10 and the subsequent parts to be replaced as required. For example, there are cases where the filter function of the hollow fiber membrane part 11 used in the filter part 9 of the posterior part 10 may deteriorate over time as the protein or other substances contained in the aqueous humor clogs the hollow fiber membrane. In this case, the posterior part 10, including the filter part 9, can be replaced with a new replacement part by detaching the posterior part 10 at the joints 5 and 6. In this way, the intraocular pressure reducing effect can be sustained for extended periods of time.

Further, because only the posterior part 10 is replaced, the cost of replacement is much cheaper than the case where the aqueous humor drainage implant 1 needs to be re-installed entirely. In addition, the physical pain the patient must endure is greatly relieved. Further, because the joints 5 and 6 are positioned external to but in contact with living tissues such as the conjunctiva 14, the upper eyelid 18, and the lower eyelid 19, it is preferable that the joints 5 and 6 be made of material with good biocompatibility and good durability. The type of material is not particularly limited as long as it has such characteristics. For example, polymer materials such as polyacetal resin, silicone resin, polyethylene resin, polypropylene resin, ethylene-vinyl alcohol copolymer, polyurethane resin, ABS (Acrylonitrile-Butadiene-Stylene) resin, and polycarbonate resin are available. In addition, ceramics such as alumina and titania, or metals such as stainless steel can also be used.

The joints 5 and 6 may have any conventional structure as long as it serves to prevent entry of foreign substances and join the first tube 3 to the second tube 7 of the posterior part 10. Examples of such structures include a tapered connector, a threaded connector, a ball joint, a coupler (the product of NITTO KOHKI CO., LTD.), and a tube fitter (the product of NITTO KOHKI CO., LTD.). Among these different structures, those employed, for example, by the coupler and tube fitter (both the products of NITTO KOHKI CO., LTD.) are particularly preferable because such structures are easily detachable and allow an operator to check whether the joints are in place by the sound or feel of clicking.

The joints 5 and 6 may be sized and shaped in any manner as long as invasiveness of the conjunctiva 14, the upper eyelid 18, and the lower eyelid 19 following eye movement is controlled. For example, the joints 5 and 6 may be sized to 1 mm³ to 5 mm³ each, and may have a curved surface as shown in Fig. 2. With the joints 5 and 6 sized and shaped this way, invasiveness to the body can be minimized.

While a representative structure and embodiment of the aqueous humor drainage implant of the present invention is described above with reference to Fig. 1 through Fig. 5, the invention is not limited in any way by the foregoing examples. It should be understood that the foregoing examples are not intended to limit the invention to the particular forms disclosed, but on the contrary, the invention is to cover all modifications, equivalents, and alternatives falling within the scope of the invention as defined in the appended claims.

An aqueous humor drainage implant of the present invention may be implemented as follows.

Specifically, an aqueous humor drainage implant may be implemented as a first aqueous humor drainage implant for draining aqueous humor from the interior of the eye to the exterior of the conjunctiva, the first aqueous humor drainage implant including a guiding tube part for guiding aqueous humor to exterior of the eye, and a filter part for preventing reflux infection from the exterior to interior of the eye, and being structured and shaped to be positioned in the eye and outside the conjunctiva with reduced invasiveness.

According to the invention, the aqueous humor drainage implant can easily be positioned with reduced invasiveness, and the aqueous humor can be drained to the exterior of the aqueous humor both safely and reliably.

The first aqueous humor drainage implant may be shaped and structured to be positioned in the lacrimal canaliculus, lacrimal sac, or nasolacrimal duct. Further, the first aqueous humor drainage implant may be shaped and structured to be positioned on the conjunctiva.

According to the invention, the aqueous humor drainage implant can be positioned on the conjunctiva, or in the lacrimal canaliculus, lacrimal sac, or nasolacrimal duct, depending on patient conditions. Further, regardless of the position, the aqueous humor drainage implant can be installed with reduced invasiveness.

The first aqueous humor drainage implant may be adapted so that the filter part uses a hollow fiber membrane for preventing reflux infection at the viral level.

According to the invention, reflux infection caused by viruses or any other pathogens can be prevented.

The first aqueous humor drainage implant may be adapted so that the filter part uses a hollow fiber membrane that has been treated to electrically block viruses or other microorganisms.

According to the invention, reflux infection at the viral level can be prevented more reliably, while draining a sufficient amount of aqueous humor to reduce intraocular pressure.

The first aqueous humor drainage implant may be adapted so that its outer surface is rendered hydrophilic.

According to the invention, the guiding tube part and the filter part can have improved biocompatibility, and the hollow fiber membrane of the filter part can drain a sufficient amount of aqueous humor to reduce intraocular pressure.

The first aqueous humor drainage implant may be adapted to optionally include a joint for enabling the filter part to be replaced.

According to the invention, the filter part can be replaced when it is deteriorated or damaged. That is, the intraocular pressure reducing effect of the aqueous humor drainage implant can be sustained for extended periods of time by a simpler, less expensive, and more patient friendly method, as compared with the case where the entire aqueous humor drainage implant is reinstalled.

The first aqueous humor drainage implant may be adapted so that a portion of the guiding tube part to be positioned outside the conjunctiva includes a tube that can easily deform according to eye movement and that is flexible enough to relieve invasiveness to the ocular tissue.

In this way, the invention can prevent problems caused by eye movement, such as invasiveness to the ocular tissue, patient's pain, and displacement of the aqueous humor drainage implant.

As described above, in the aqueous humor drainage implant for glaucoma treatment, the guiding tube part includes an eye-side guiding tube part and an outside-conjunctiva guiding tube part.

In this way, in installing the aqueous humor drainage implant for glaucoma treatment in the patient, the guiding tube part can easily be positioned with reduced invasiveness based on the anatomical structure of the eye and nearby organs.

Further, with the filter part connected to one end of the guiding tube part, reflux infection from the exterior to interior of the eye can be prevented. That is, the aqueous humor can be safely drained out of the eye to the exterior of the conjunctiva.

Further, the outside-conjunctiva guiding tube part may have an outer diameter smaller than an inner diameter of the nasolacrimal duct. In this way, with the guiding tube positioned in the lacrimal passage including the lacrimal canaliculus, lacrimal sac, and nasolacrimal duct, the aqueous humor can drain into the nasal cavity through the lacrimal passage. That is, the guiding tube can be positioned with reduced invasiveness.

Further, the outside-conjunctiva guiding tube part and the filter part may be shaped to have a curved outer surface and may be sized to have substantially the same outer diameter. In this way, the outside-conjunctiva guiding tube part and the filter part can be easily positioned along the eye wall. Further, damage to the conjunctiva or the sense of foreign object can be relieved.

Further, the filter part may include a hollow fiber membrane made of at least one kind of polymer material selected from the group consisting of a polyolefin polymer, a polyvinyl alcohol polymer, an ethylene-vinyl alcohol copolymer, a polysulfone polymer, a polyacrylonitrile polymer, a cellulose polymer, cellulose acetate polymer, a polymethyl methacrylate polymer, and a polyamide polymer. Further, it is preferable that the hollow fiber membrane have an average pore diameter of no greater than 0.3 µm, or more preferably no greater than 0.02 µm. In this way, the hollow fiber membrane can prevent reflux infection at the viral level.

Further, the filter part may include a chemically bound anionic group or cationic group. In this way, the reflux infection at the viral level can be prevented more reliably, and a sufficient amount of aqueous humor can be drained to reduce intraocular pressure.

Further, the guiding tube part and the filter part may be rendered hydrophilic. In this way, the guiding tube part and the filter part can have improved biocompatibility, and the filter part can stably drain a sufficient amount of aqueous humor necessary to reduce intraocular pressure.

The aqueous humor drainage implant for glaucoma treatment may be adapted to further include a joint part for detachably connecting the eye-side guiding tube part and the outside-conjunctiva guiding tube part. In this way, the intraocular pressure relieving effect of the aqueous humor drainage implant can be sustained for extended periods of time by a simpler, less expensive, and more patient friendly method.

Further, the outside-conjunctiva guiding tube part may have a flexural modulus of no greater than 2000 Mpa at ordinary temperature. Further, the outside-conjunctiva guiding tube part may include an outside-conjunctiva eye-side guiding tube part and an outside-conjunctiva filter-side guiding tube part, and the outside-conjunctiva eye-side guiding tube part and the outside-conjunctiva filter-side guiding tube part may be connected to each other, wherein the outside-conjunctiva eye-side guiding tube part has a smaller flexural modulus than the outside-conjunctiva filter-side guiding tube part at ordinary temperature.

In this way, the invention prevents problems caused by eye movement, such as invasiveness to the ocular tissue, patient's pain, and displacement of the aqueous humor drainage implant.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, the aqueous humor drainage implant for glaucoma treatment can be positioned in the eye and outside the conjunctiva with reduced invasiveness, and is therefore useful as a device for the treatment of glaucoma.

## Claims

1. An aqueous humor drainage implant for draining aqueous humor in an eye to exterior of the conjunctiva for glaucoma treatment, comprising:
a guiding tube part for guiding the aqueous humor to exterior of the eye; and
a filter part, connected to one end of the guiding tube part, for preventing reflux infection from the exterior to interior of the eye,
wherein the guiding tube part includes an eye-side guiding part and an outside-conjunctiva guiding tube part.

2. An aqueous humor drainage implant for glaucoma treatment as set forth in claim 1, wherein the outside-conjunctiva guiding tube part has an outer diameter smaller than an inner diameter of the nasolacrimal duct.

3. An aqueous humor drainage implant for glaucoma treatment as set forth in claim 1, wherein the outside-conjunctiva guiding tube part and the filter part are shaped to have a curved outer surface and sized to have substantially the same outer diameter.

4. An aqueous humor drainage implant for glaucoma treatment as set forth in claim 1, wherein the filter part includes a chemically bound anionic group or cationic group.

5. An aqueous humor drainage implant for glaucoma treatment as set forth in claim 1, wherein the guiding tube part and the filter part are rendered hydrophilic.

6. An aqueous humor drainage implant for glaucoma treatment as set forth in claim 1, further comprising a joint part for detachably connecting the eye-side guiding tube part and the outside-conjunctiva guiding tube part.

7. An aqueous humor drainage implant for glaucoma treatment as set forth in claim 1, wherein the outside-conjunctiva guiding tube part has a flexural modulus of no greater than 2000 Mpa at ordinary temperature.

8. An aqueous humor drainage implant for glaucoma treatment as set forth in claim 1,
wherein the outside-conjunctiva guiding tube part includes an outside-conjunctiva eye-side guiding tube part and an outside-conjunctiva filter-side guiding tube part, wherein the outside-conjunctiva eye-side guiding tube part and the outside-conjunctiva filter-side guiding tube part are connected to each other, and wherein the outside-conjunctiva eye-side guiding tube part has a smaller flexural modulus than the outside-conjunctiva filter-side guiding tube part at ordinary temperature.

9. An aqueous humor drainage implant for glaucoma treatment as set forth in claim 1, wherein the filter part includes a hollow fiber membrane made of at least one kind of polymer material selected from the group consisting of a polyolefin polymer, a polyvinyl alcohol polymer, an ethylene-vinyl alcohol copolymer, a polysulfone polymer, a polyacrylonitrile polymer, a cellulose polymer, cellulose acetate polymer, a polymethyl methacrylate polymer, and a polyamide polymer.

10. An aqueous humor drainage implant for glaucoma treatment as set forth in claim 9, wherein the hollow fiber membrane has an average pore diameter of no greater than 0.3 µm.

11. An aqueous humor drainage implant for glaucoma treatment as set forth in claim 9, wherein the hollow fiber membrane has an average pore diameter of no greater than 0.02 µm.
